# DEMANDE DE BREVET EUROPEEN

(11) **EP 1 797 864 A1**
(43) Date de publication de la demande: **20.06.2007**
(21) Numéro de dépôt: 06301239.7
(22) Date de dépôt: 12.12.2006
(51) Int. Cl.: A61K 8/42, A61Q 1/10

(54) **Composition cosmétique texturée par un dérivé bis-urée asymétrique spécifique**

(30) Priorité: 13.12.2005 FR 0553849
(71) Demandeur: L'ORÉAL, 75008 Paris (FR)
(72) Inventeur: Chodorowski-Kimmes, Sandrine, 60300 Senlis (FR); Baghdadli, Nawel, 91300 Massy (FR); Arnaud-Roux, Mireille, 93600 Aulnay-sous-Bois (FR); Garipova, Goulnara, 45380 La Chapelle Saint Mesmin (FR); Bouteiller, Laurent, 92340 Bourg La Reine (FR)
(74) Mandataire: Le Coupanec, Pascale A.M.P.

(57) **Abrégé**

La présente invention concerne une composition cosmétique à phase grasse liquide, comprenant dans un milieu physiologiquement acceptable au moins une phase grasse liquide, dans laquelle ladite phase est texturée par une quantité efficace d'au moins un composé de bis-urée asymétrique de formule définie.

## Description

La présente invention concerne le domaine cosmétique et plus particulièrement une composition cosmétique à phase grasse liquide texturée par un composé bis-urée asymétrique.

Pour structurer les huiles et leur donner la texture ou la viscosité souhaitée, l'utilisation d'organogélateurs est bien connue de l'homme de l'art. Les organogélateurs modifient les interactions moléculaires à l'intérieur de l'huile et changent ses caractéristiques physiques et/ou chimiques. Toutefois, la solubilisation de ces molécules organogélatrices dans une huile ou un mélange d'huiles requiert une température souvent élevée, ce qui peut générer des coûts supplémentaires de chauffage et surtout être incompatible avec la présence de molécules thermosensibles. De plus, le gel obtenu dans ces conditions ne présent pas toujours la stabilité nécessaire au cours du temps.

Il est connu de l'homme de l'art que les composés contenant des urées et plus particulièrement des bis-urées sont de bons organogélateurs. Nous pouvons par exemple citer Feringa et coll. dans Chem.Eur.J, 1997, 3,1238-1243, ou encore Hanabusa et coll. dans Chem. Lett., 1996, 885-886 décrivant des bis-urées comme gélateurs de solvants organiques. De même Andrew D. Hamilton décrit dans Tetahedron Letters 39 (1998) 7447-7450, des organogelateurs à base de molécules bis-urées capables de gélifier différents solvants organiques dont l'acétate d'éthyle.

L'utilisation de certains bis-urées symétriques et asymétriques à titre d'organogélateur a également été envisagée dans les documents WO 02/47628 et JP 2003-064346. Ces documents décrivent entre autre l'utilisation de bis-urées symétriques et/ou asymétriques pour gélifier des milieux cosmétiques ou non. Toutefois, les bis-urées symétriques et asymétriques décrites dans ces documents ne se solubilisent pas toutes à température ambiante et/ou dans toutes les huiles cosmétiques. Pour identifier un agent gélifiant capable de gélifier de manière satisfaisante une composition cosmétique particulière et apte à être solubilisé à température ambiante dans les huiles de la composition, de nombreux tests doivent être réalisés au préalable.

Il existe donc un besoin d'une formule de molécules organogélatrices que l'on pourrait qualifier d'universelle dans la mesure où elle réunit des molécules de structure chimique proche, qui s'avèrent respectivement efficaces pour gélifier, à température ambiante, au moins une et avantageusement plusieurs huiles cosmétiques différentes.

La présente invention a précisément pour objectif de proposer de nouvelles bis-urées asymétriques conformes à ces exigences.

En conséquence, selon l'un de ses aspects, l'invention concerne une composition cosmétique comprenant dans un milieu physiologiquement acceptable au moins une phase grasse liquide, caractérisée en ce que ladite phase est texturée par une quantité efficace d'au moins un composé de formule (I) : 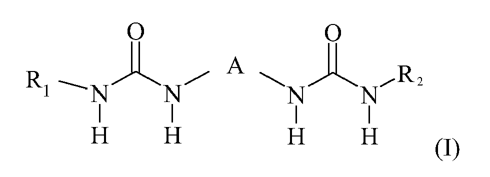 dans laquelle A, R₁ et R₂ sont tels que définis ci-après.

De manière inattendue, les inventeurs ont découvert que les composés de bis-urée asymétriques considérés selon l'invention étaient des agents gélifiants de choix pour répondre au besoin exprimé précédemment.

Selon un autre de ses aspects, l'invention concerne l'utilisation d'au moins un composé de bis-urée asymétrique tel que décrit précédemment pour structurer une composition cosmétique.

Selon encore un autre de ses aspects, l'invention concerne un procédé cosmétique c'est-à-dire non thérapeutique de maquillage et/ou de soin d'une matière kératinique, notamment de la peau du corps ou du visage, des ongles, des cils et des cheveux comprenant l'application sur la surface à traiter d'une composition cosmétique comprenant au moins un composé de bis-urée asymétrique tel que décrit précédemment.

### BIS-UREES

Comme précisé ci-dessus, les composés de bis-urée plus particulièrement considérés selon l'invention répondent à la formule générale (I) suivante : 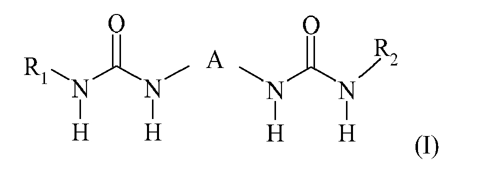 dans laquelle :
- A est un groupement de formule : 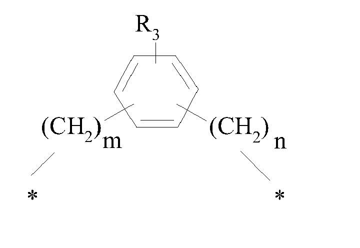 avec
   - R₃ étant un atome d'hydrogène ou un radical alkyle en C₁ à C₄ linéaire ou ramifié,
   - n et m étant, indépendamment l'un de l'autre, égaux à 0 ou 1, et
   - * symbolisant le point de rattachement du groupement A aux deux atomes d'azote du reste du composé de formule générale (I),
- R₁ est un radical carboné en C₃ à C₁₅, ramifié, non cyclique, saturé ou insaturé et contenant éventuellement de 1 à 3 hétéroatomes choisis parmi O, S, F et N et/ou un carbonyle, et leurs combinaisons,
- R₂ est différent de R₁ et est choisi parmi les radicaux alkyles en C₁-C₂₄, saturé ou insaturé, linéaire, ramifié ou cyclique, contenant éventuellement de 1 à 3 hétéroatomes choisis parmi O, S, F et N, et éventuellement substitué par :
   - 1, 2 ou 3 radicaux hydroxy,
   - un radical ester (-COOR₄), avec R₄ étant un radical alkyle, linéaire
   ou ramifié, ayant de 1 à 8, notamment 1 à 6, voire 2 à 4 atomes de carbone ;
   - un radical cyclique saturé, insaturé ou aromatique ayant de 5 à 12 atomes de carbone, en particulier un radical phényle éventuellement substitué par un ou plusieurs radicaux identiques ou différents choisis parmi les radicaux alkyle en C₁-C₄, trifluorométhyle, ou un dérivé morpholine, et/ou
   - un ou plusieurs radicaux alkyles linéaires ou ramifiés en C₁-C₄,
   ou l'un de ses sels ou isomères.

En particulier n et m sont égaux, et plus particulièrement égaux à zéro et R₃ est un radical R'₃, tel que défini ci-après. Ainsi, de manière préférée, A représente un groupement

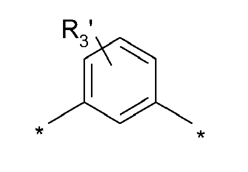

Avec R₃' étant un radical alkyle en C₁ à C₄ linéaire ou ramifié et * symbolisant les point de rattachement du groupement A aux deux atomes d'azote du reste du composé de formule générale (I).

Selon une variante de l'invention, le composé de formule générale (I) comprend à titre de A, au moins un groupement choisi parmi : 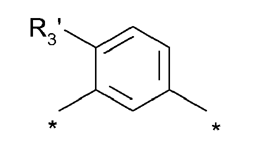 ou 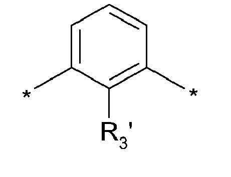 avec R₃' et * étant tels que défini précédemment.

En particulier, R₃' peut être un groupement méthyle, et dans ce cas le groupement A représente un groupement 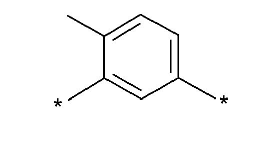 ou 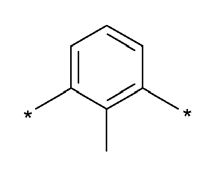 * étant tel que défini précédemment.

En particulier, les composés sont tels que A est un mélange de 2,4-tolylène et 2,6-tolylène notamment dans des proportions (isomère 2,4)/(isomère 2,6) variant de 95/5 à 80/20.

Selon un mode de réalisation de l'invention, le composé de formule générale (I) comprend à titre de R₁, un radical en C₆-C₁₅ ramifié.

Selon un mode de réalisation de l'invention, le composé de formule générale (I) comprend à titre de R₁, un groupement choisi parmi : 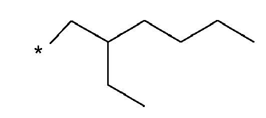 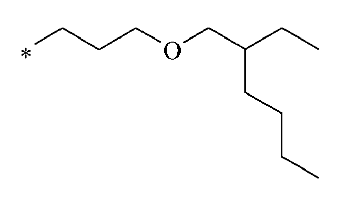 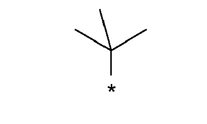 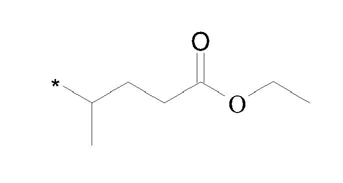 avec * symbolisant le point de rattachement du groupement R₁ à l'azote du reste du composé de formule générale (I).

Comme il ressort des exemples ci-après, la présence de l'un et/ou l'autre de ses deux radicaux dans la molécule de formule générale (I) s'avère particulièrement avantageuse pour conférer un caractère universel au sens de l'invention, aux dérivés bis-urée asymétriques correspondants.

En ce qui concerne R₂ qui est différent de R₁, il peut être avantageusement choisi parmi les groupements suivants : 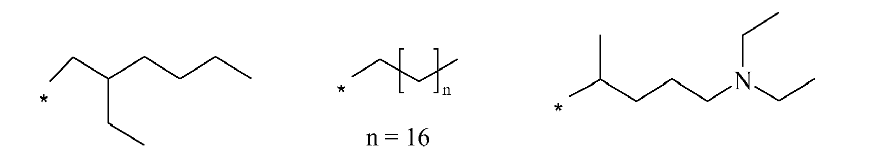 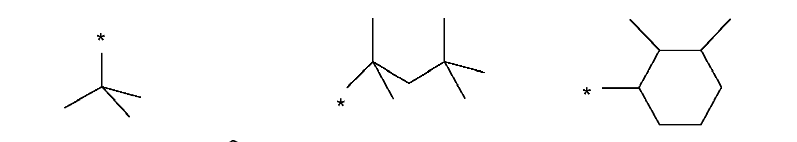 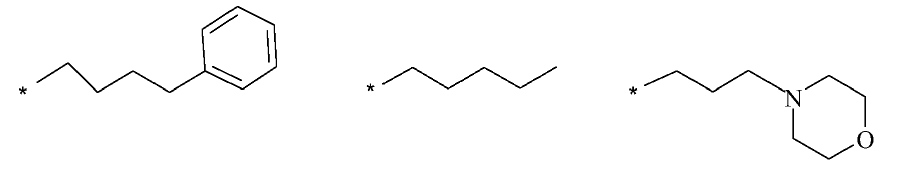 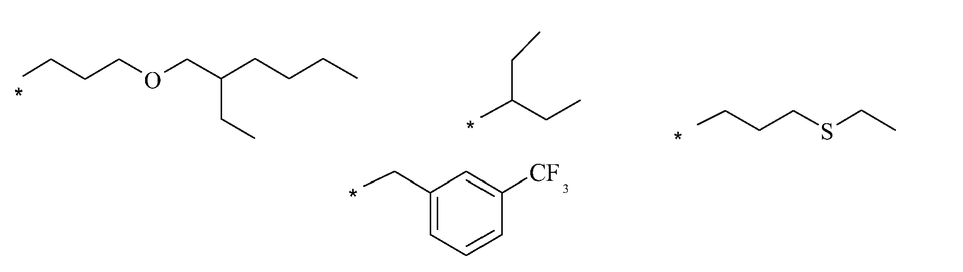 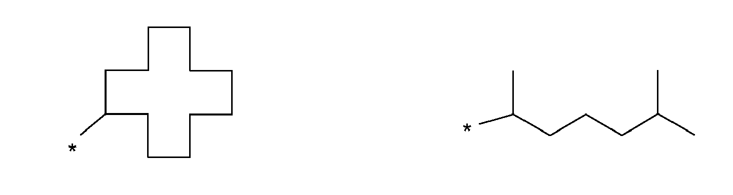 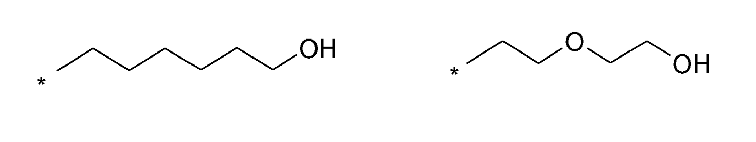 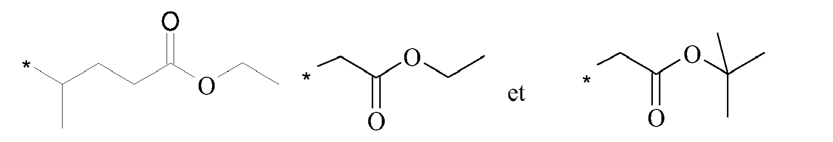 avec * symbolisant le point de rattachement du groupement R₂ à l'azote du reste du composé de formule générale (I).

A titre représentatif et non limitatif des composés convenant tout particulièrement à l'invention, on peut plus particulièrement citer les composés suivants : 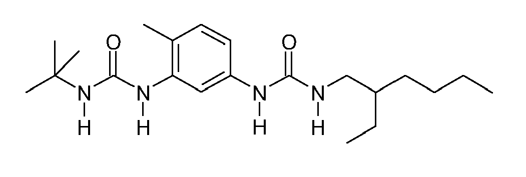 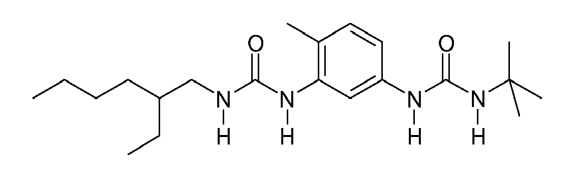 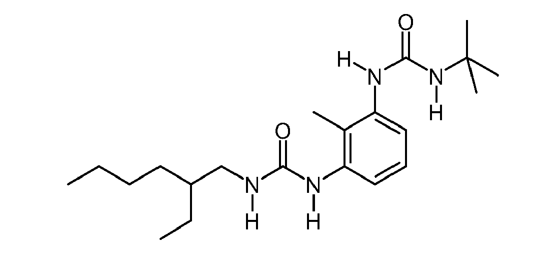 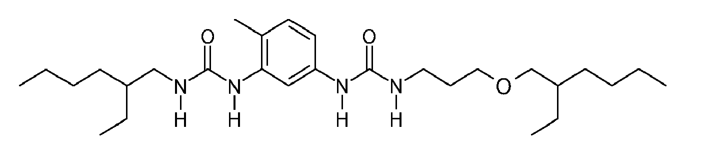 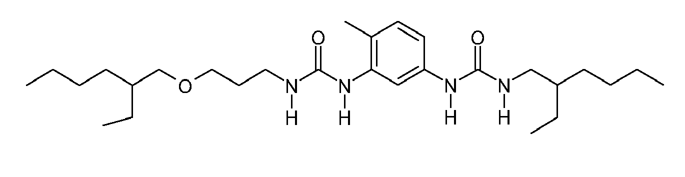 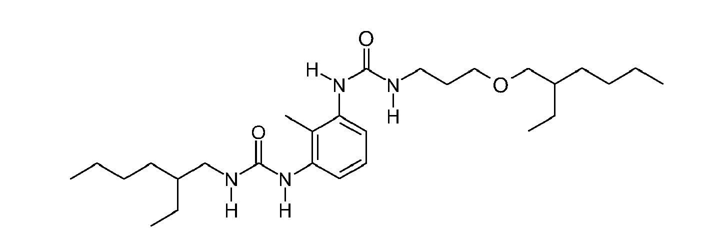 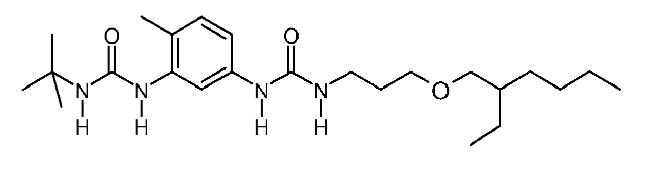 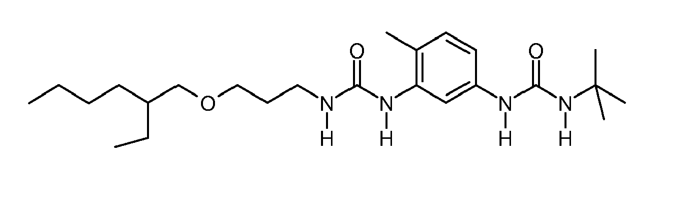 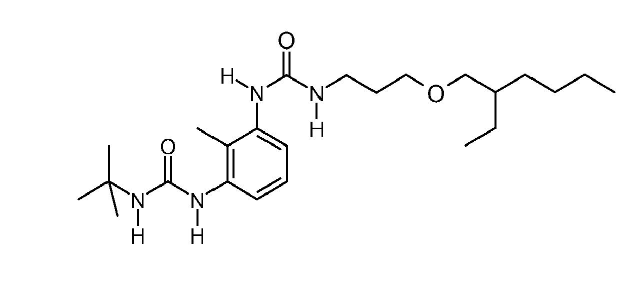 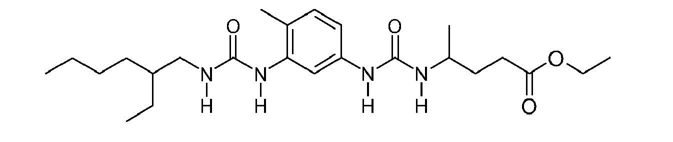 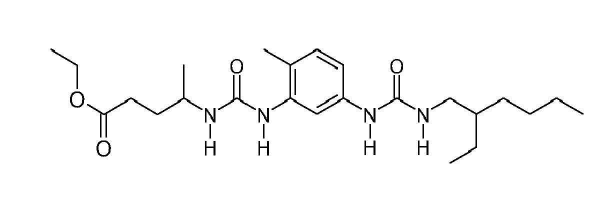 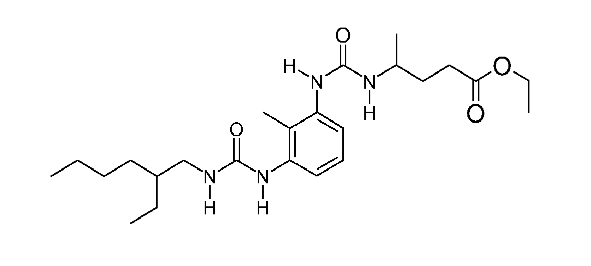 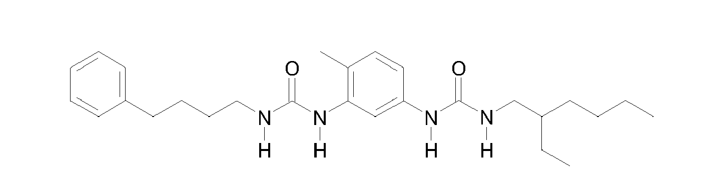 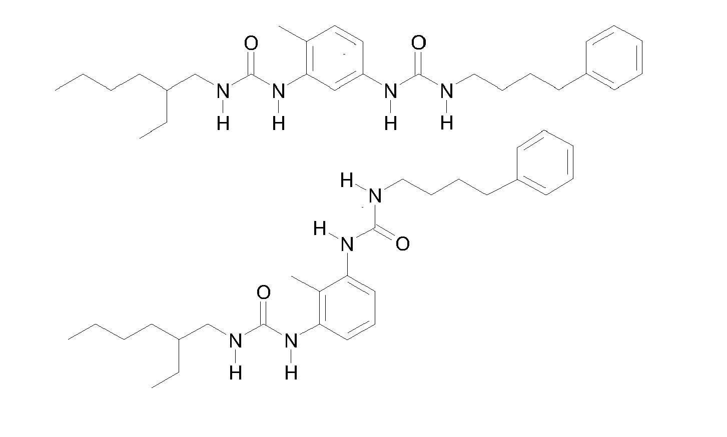 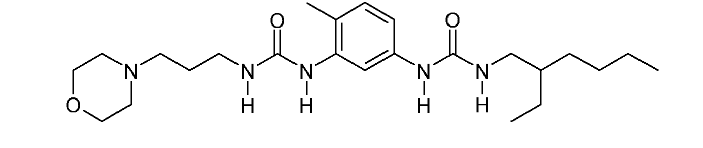 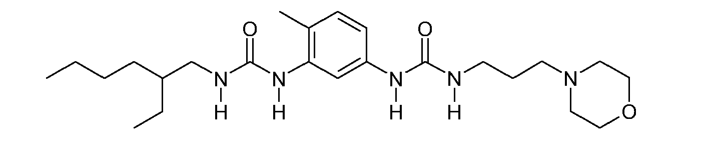 et

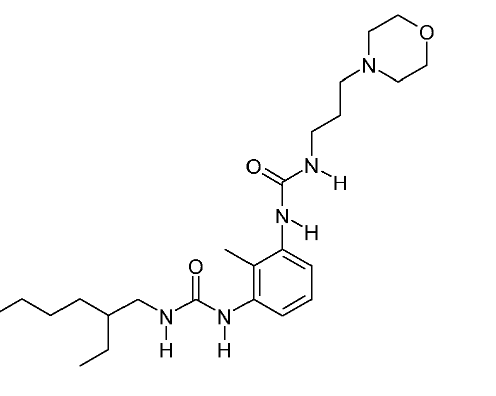

Par « conviennent particulièrement dans le cadre de l'invention », on entend que le composé de formule générale (I), seul ou en mélange en différentes proportions, peut se solubiliser à température ambiante dans plusieurs huiles cosmétiques et qu'il s'avère efficace pour gélifier ladite huile ou mélange d'huiles considéré(e) et donc lui conférer les propriétés physiques et/ou chimiques souhaitées.

Au sens de la présente invention, le terme « quantité efficace » désigne la quantité nécessaire et suffisante pour obtenir la texturation de l'huile ou mélange d'huiles considéré dans la composition selon l'invention.

Cette texturation se traduit en particulier par une augmentation de la viscosité qui peut être due à l'introduction d'au moins un composé de formule générale (I).

Par exemple, les compositions selon l'invention peuvent contenir de 0,0001 à 5 % en poids de bis-urée asymétrique, notamment de 0,001 à 1 %, voir de 0,004 à 0,5 % en poids d'au moins une bis-urée asymétrique par rapport au poids total de la phase grasse liquide

Pour des raisons évidentes, cette quantité efficace est susceptible de varier significativement selon d'une part la nature des substituants R₁ et/ou R₂ du dérivé bis-urée, l'isomère de position, le fait qu'il est utilisé à l'état pur ou en mélange avec d'autres dérivés bis-urée de formule (I) ou non, et d'autre part la nature de la phase huileuse.

Pour des raisons notamment liées à la procédure de préparation, les dérivés bis-urées asymétriques de formule générale (I), peuvent être mis en oeuvre dans le cadre de la présente invention sous la forme d'un mélange de dérivés de formule (I) entre eux, et/ou avec les deux formes de dérivés bis-urées symétriques correspondantes. On entend au sens de la présente invention par bis-urées symétriques, les bis-urées selon la formule (I) avec des radicaux R₁ et R₂ identiques.

D'une manière générale, le composé de formule générale (I) selon l'invention dérive de la réaction entre au moins un diisocyanate de formule : 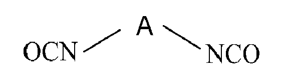 et au moins deux amines primaires distinctes de formule : 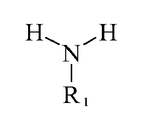 et 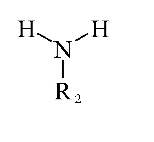 avec A, R₁ et R₂ tels que définis précédemment.

Le cas échéant, les différents diisocyanates peuvent être des isomères de positions du susbtituant R₃ sur le groupement A, notamment dans des proportions 95/5 ou 80/20.

Par au moins deux amines primaires distinctes, on entend qu'on peut y adjoindre d'autres amines primaires Rₓ-NH₂ dans lesquelles Rₓ répond aux définitions proposées pour R₁ et R₂.

Le nombre d'amines utilisées pour la réaction peut être supérieur ou égal à 2, par exemple peut varier de 2 à 20, et plus particulièrement entre 3 et 10. Pour faciliter la préparation de la composition et la caractérisation du mélange ainsi obtenu, il est avantageux de se limiter à l'utilisation de deux amines.

De préférence l'ensemble des amines utilisées est dans un rapport molaire de 2 à 3 équivalents, notamment 2,1 à 2,5, voire 2,2 équivalents pour un équivalent de diisocyanate(s).

Dans le cas particulier où seulement deux amines primaires sont utilisées pour la réaction, le rapport molaire n(R₁)/n(R₂) peut être compris entre 1/99 et 99/1, plus particulièrement entre 5/95 et 95/5, voire entre 10/90 et 90/10 avec n(R₁) correspondant au nombre de moles de : 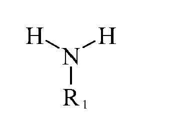 et n(R₂) correspondant au nombre de moles de : 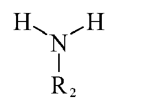 et avec R₁, R₂, tels que définis précédemment.

La réaction est généralement effectuée sous atmosphère inerte par exemple sous argon, en milieu anhydre avec par exemple une température du milieu réactionnel qui est maintenue inférieure à 50 °C, voire comprise entre 15 °C et 40 °C, de préférence entre 18 °C et 25 °C.

Le(s) diisocyanate(s) peut(peuvent) être mis en solution dans un solvant anhydre tel que la tétrahydrofurane, la 2-méthyl-tétrahydrofurane, la N-méthylpyrrolidone, l'acétate de butyle ou encore la méthyl-éthylcétone à une concentration pouvant aller de 1 à 30 % en poids, de préférence de 2 à 20 %, voire de 4 à 10 % en poids.

Une solution contenant les amines est généralement préparée dans le même solvant que le(s) diisocyanate(s) à une concentration allant par exemple de 0,1 à 99,9 % en masse. La température du milieu réactionnel ne doit préférentiellement pas dépasser 40 °C et la concentration des amines ainsi que la vitesse d'addition de la solution contenant les amines doivent donc être préférentiellement ajustées à cette nécessité. Le milieu réactionnel peut être laissé sous agitation par exemple pendant 30 min à 12 heures. Le contrôle de l'avancement de la réaction peut être réalisé par spectrométrie infra-rouge (notamment en observant la disparition de la bande NCO entre 2250 et 2280 cm⁻¹). Par exemple, en fin de réaction, on verse le milieu réactionnel dans une grande quantité d'eau acide (notamment un pH 3-4 par HCl). On obtient alors un précipité qui est filtré, lavé par exemple plusieurs fois notamment à l'eau et séché sous pression réduite, notamment sous vide ou lyophilisé.

En conséquence, à l'issue de cette réaction, on obtient conjointement avec le dérivé asymétrique de formule (I) attendu, au moins l'une des deux formes suivantes : 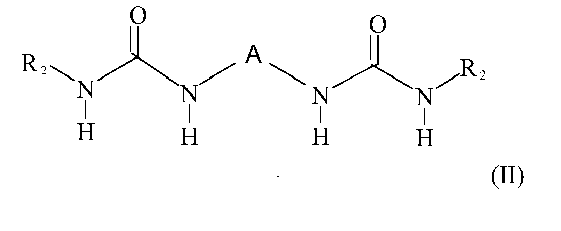 et 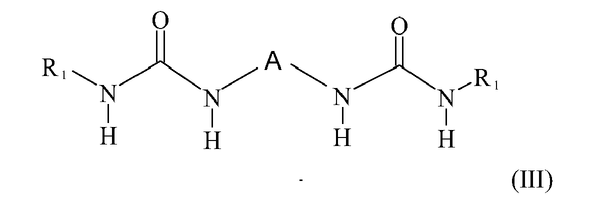 avec A, R₁ et R₂ tels que définis précédemment, avec A étant identique dans les formules (I), (II), (III), R₁ étant identique en formules (I) et (III) et R₂ étant identique en formules (I) et (II).

Le précipité correspond aux composés de formule (I) attendu(s), et peut être caractérisé par spectrométrie RMN (¹H et ou ¹³C) et/ou par HPLC et peut être utilisé tel quel pour la texturation du milieu huileux considéré.

En fin de réaction, le mélange de bis-urées (I), (II) et (III) est isolé et peut être utilisé tel quel pour la gélification du milieu huileux souhaité.

Auquel cas, le composé de formule (I) est mis en oeuvre dans la phase grasse liquide sous la forme d'un mélange avec les composés de formule générale (II) et (III).

Le mélange de bis-urées est avantageusement soluble à une température inférieure ou égale à 50 °C, voire inférieure ou égale à 30 °C, et notamment à température ambiante, dans la phase grasse liquide à texturer.

### PHASE GRASSE LIQUIDE

Les compositions selon l'invention peuvent comprendre une phase huileuse comprenant par exemple des huiles non siliconées et/ou des huiles siliconées.

Par phase grasse liquide, au sens de la demande, on entend une phase grasse liquide à température ambiante (25 °C) et pression atmosphérique (760 mm de Hg), composée d'un ou plusieurs corps gras liquides à température ambiante, appelés aussi huiles, compatibles entre eux.

La phase huileuse peut représenter de 1 à 99,99 % en poids de la composition, voire de 10 à 90 %, en particulier de 20 à 50 % en poids de la composition.

La phase grasse liquide peut contenir des huiles aptes ou non à solubiliser ou à être gélifiées par un composé bis-urée tel que défini précédemment. L'homme du métier veillera à choisir les huiles et leurs proportions respectives dans la phase grasse liquide afin d'obtenir une solubilisation du bisurée et une gélification de la phase grasse liquide telles que souhaitées.

### a. Huile siliconée

Selon une variante de l'invention, la phase grasse liquide comprend au moins une huile siliconée.

Les huiles siliconées utilisables dans la phase grasse liquide de l'invention peuvent également être des polydiméthylsiloxanes (PDMS) non volatils, linéaires, liquides à température ambiante ; les polyalkylméthylsiloxanes et en particulier des polydiméthylsiloxanes comportant des groupements alkyle, alcoxy ou phényle, pendants et/ou en bout de chaîne siliconée, groupements ayant chacun de 2 à 24 atomes de carbone ; les diméthicone copolyols, les alkylméthicone copolyols ; des silicones phénylées comme les phényl triméthicones, des phényl diméthicones, des phényl triméthylsiloxy diphénylsiloxanes, des diphényl diméthicones, des diphényl méthyldiphényl trisiloxanes, des 2-phényléthyl triméthylsiloxysilicates, la cétyldiméthicone, les silicones à groupes alkylglycéryl éthers, les silicones à groupes amines latéraux et le dilauroyltriméthylol propane siloxysilicate ; des silicones fluorées à groupement(s) pendant(s) ou en bout de chaîne ayant de 1 à 12 atomes de carbone dont tout ou partie des atomes d'hydrogène est substitué par des atomes de fluor telles que les silicones à groupes alkyle et perfluoralkyle, les silicones à groupes latéraux oxyéthylénés/oxypropylénés (OE/PP) et à groupes perfluorés, les silicones à groupes latéraux perfluorés et à groupes latéraux glycérolés, et les perfluoroalkylméthylphénylsiloxanes, et leurs mélanges.

De préférence, l'huile siliconée peut être choisie parmi les silicones cycliques volatiles telles que D4, D5 ou D6, et les phényltriméthicones, et leurs mélanges.

La phase grasse liquide contient avantageusement au moins 5 %, par exemple de 10 à 90 % en poids d'huile(s) siliconée(s).

### b. Huile ester

Selon une variante de l'invention, au moins une des huiles de la phase grasse liquide est une huile, dite "huile ester", qui est choisie parmi les esters des acides monocarboxyliques avec les monoalcools et polyalcools.

Avantageusement, ledit ester répond à la formule suivante (IV) :

R₁-CO-O-R₂ (IV)

où R₁ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par "éventuellement substitué", on entend que R₁ et ou R₂ peuvent porter un ou plusieurs substituants choisis, par exemple, parmi les groupements comprenant un ou plusieurs hétéroatomes choisi parmi O, N et S, tels que amino, amine, alcoxy, hydroxyle.

Le nombre d'atomes de carbone de R₁ + R₂ peut être supérieur ou égal à 9.

Des exemples des groupes R₁ sont ceux dérivés des acides gras de préférence supérieure choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.

Des exemples d'esters utilisables dans les phases grasses des compositions de l'invention sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

De préférence, R₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone. On peut également citer les esters comprenant 3 à 10 atomes de carbone au total, tels que les acétates d'éthyle et/ou de butyle.

Le N-lauroyl sarosinate d'isopropyle (Eldew 205-SL d'Ajinomoto) peut être également avantageux selon un aspect de l'invention.

Avantageusement, la phase grasse liquide comprend de 0,5 à 100 % en poids d'huile(s) ester(s), de préférence de 1 à 80 % en poids, de préférence encore de 2 à 50 % poids, mieux de 3 à 40 % en poids.

### c. Huile non siliconée

La phase grasse liquide des compositions selon l'invention peut contenir en outre une ou plusieurs huiles non siliconées distinctes des huiles esters décrites précédemment. Ces huiles non siliconées peuvent être choisies dans le groupe des huiles hydrocarbonées et des éthers volatils.

L'huile non siliconée peut aussi être choisie parmi les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés.

La phase grasse liquide peut aussi contenir d'autres huiles non siliconées, par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel,
- les éthers de synthèse en C₆-C₄₀,
- les alcools gras en C₈-C₃₂ comme l'alcool oléique ou l'octyldodécanol,
- les acides gras en C₈-C₃₂, comme l'acide oléique, linoléique ou linolénique, et
- leurs mélanges.

La phase grasse liquide peut encore contenir des huiles apolaires telles que les hydrocarbures ou fluorocarbures, linéaires ou ramifiés, d'origine synthétique ou minérale, comme les huiles de paraffine (telles que les isoparaffines en C₈-C₁₆, l'isododécane, l'isohéxadécane) et ses dérivés, la vaseline, les polydécènes, les polyisobutènes hydrogénés tel que le parléam, le squalane, et leurs mélanges.

Tout particulièrement, la phase grasse liquide comprend au moins un composé lipophile choisi dans la liste ci-après :
- les monoalcools en C₆-C₃₂, de préférence C₈-C₂₈, encore mieux C₁₂-C₂₆, et préférentiellement l'octyldodécanol ;
- les alcanes ramifiés en C₆-C₃₂, de préférence C₈-C₂₈, encore mieux C₁₂-C₂₆, et préférentiellement le parléam de formule -(CH₂-CH(CH₃)n- avec n=4 à 8, et l'isododécane ;
- les alcanes linéaires en C₁₃-C₄₈, de préférence C₁₈-C₄₀, encore mieux C₂₀-C₃₂;
- les huiles bifonctionnelles, comprenant deux fonctions choisies parmi ester et/ou amide, comprenant 6 à 30 atomes de carbone, notamment 8 à 28, encore mieux 10 à 24 carbones, et 4 hétéroatomes choisis parmi O et N ; de préférence les fonctions amide et ester sont dans la chaîne ; et
- leurs mélanges.

Généralement, les huiles non siliconées représentent de 5 à 95 % du poids total de la phase grasse liquide et mieux de 20 à 75 %.

On peut bien évidemment utiliser un mélange d'huiles siliconées et d'huiles carbonées, et en particulier un mélange d'au moins un composé lipophile choisi dans la liste ci-dessus avec une huile siliconée (phényltriméthicone, PDMS, silicones volatiles).

### AUTRES CORPS GRAS

Les compositions selon l'invention peuvent en outre comprendre au moins un corps gras solide, qui peut être choisi parmi les cires et les composés pâteux.

Par cire au sens de la présente invention, on entend un composé lipophile, solide à température ambiante (25 °C), à changement d'état solide/liquide réversible, ayant un point de fusion supérieur ou égal à 30 °C, mieux supérieur à 45 °C, et pouvant aller jusqu'à 120 °C.

Les cires peuvent être hydrocarbonées, fluorées et/ou siliconées et être d'origine végétale, minérale, animale et/ou synthétique.

Comme cire utilisable dans la première composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxydiméticone ayant de 16 à 45 atomes de carbone.

Les compositions peuvent également contenir une cire micronisée, appelée également micro cire.

A titre indicatif, les compositions selon l'invention peuvent contenir de 0,1 à 50 % en poids et mieux de 1 à 30 % en poids de cire par rapport à leur poids total.

Plus particulièrement, les compositions selon l'invention peuvent comprendre de 0,1 % à 40 % en poids, notamment de 0,1 % à 30 % en poids, et plus particulièrement de 0,5 % à 25 % en poids de corps gras solide(s) par rapport au poids total de la composition.

### PHASE AQUEUSE

La composition peut le cas échéant comprendre au moins une phase aqueuse qui peut ou non être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄ et les aldéhydes en C₂-C₄.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids, par rapport au poids total de la composition.

Selon un autre mode de réalisation conforme à l'invention, la réalisation est exempte d'eau.

### MATIÈRES COLORANTES

Selon un mode de réalisation, la composition selon l'invention peut contenir en outre au moins une matière colorante, organique ou inorganique, notamment de type pigments ou nacres.

Selon un autre mode de réalisation, la composition selon l'invention peut contenir en outre au moins une matière colorante choisie parmi les colorants lipophiles, les colorants hydrophiles, les pigments, les nacres, les matériaux à effet optique spécifique, et leurs mélanges.

Par "nacres", il faut comprendre des particules colorées de toute forme, irisées
ou non, notamment produites par certains mollusques dans leur coquille ou bien synthétisées et qui présentent un effet de couleur par interférence optique.

Cette matière colorante peut être présente à raison de 0,01 à 50 % en poids par rapport au poids total de la composition, en particulier de 0,5 à 40 % et plus particulièrement de 5 à 25 %, et notamment de 0,01 à 20 %, et en particulier de 0,1 à 10 %, voire de 2 à 5 % en poids par rapport au poids total de la composition.

### AUTRES ADDITIFS

La composition de l'invention peut comprendre, en outre, tout ingrédient usuellement utilisé dans le domaine concerné.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

On peut notamment citer les actifs cosmétiques ou dermatologiques, les charges, les vitamines, les épaississants distincts de ceux répondant à la formule générale (I), les gélifiants distincts de ceux répondant à la formule générale (I), les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de stick ou bâton.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

La composition selon l'invention peut être une composition de protection ou de soin de la peau du visage, du cou, des mains ou du corps, notamment une composition antirides, anti-fatigue permettant de donner un coup d'éclat à la peau, une composition hydratante ou traitante; une composition anti-solaire ou de bronzage artificiel.

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Bien entendu, la composition de l'invention doit être cosmétiquement ou dermatologiquement acceptable, à savoir contenir un milieu physiologiquement acceptable non toxique et susceptible d'être appliqué sur la peau, les phanères ou les lèvres d'êtres humains. Par cosmétiquement acceptable, on entend au sens de l'invention une composition d'aspect, d'odeur et de toucher agréables.

La composition selon l'invention peut être fabriquée par les procédés connus, généralement utilisés dans le domaine cosmétique ou dermatologique.

L'invention a aussi pour objet un procédé de traitement cosmétique, notamment de maquillage, de nettoyage, de protection solaire, de mise en forme, de coloration, ou de soin, des matières kératiniques, notamment de la peau du corps ou du visage, des ongles, des cheveux et/ou des cils, comprenant l'application sur la surface à traiter d'une composition cosmétique telle que définie précédemment.

Les exemples figurant ci-après sont produits à titre illustratif et non limitatif du domaine de l'invention.

### MATERIEL ET METHODE

Sans indication contraire, les composés de formule générale (I) considérés dans chacun des exemples ci-après ont été préparés selon le même mode opératoire tel que décrit ci-après.

Les quantités spécifiques retenues pour les réactifs sont précisées dans les exemples ci-après.

A une solution de THF anhydre de diisocyanate de toluène est ajoutée une solution de THF anhydre du mélange d'amine. L'addition se fait sous argon, à température ambiante, la vitesse d'addition étant telle que la température du milieu réactionnel ne dépasse jamais 50°C, et plus préférentiellement 40°C. La disparition des isocyanates est suivie en spectrométrie IR par la disparition de la bande entre 2250 et 2280 cm⁻¹. Une fois que le diisocyanate a complètement réagi, le mélange réactionnel est additionné à de l'eau acidifiée à un pH3, avec de l'acide chlorhydrique. Le précipité obtenu est filtré, lavé plusieurs fois à l'eau et enfin séché sous vide ou lyophilisé. Une poudre blanche est obtenue et utilisée telle quelle après analyse (HPLC couplée à la masse) avec un rendement molaire allant de 80 à 99 %.

Les exemples figurant ci-après différent par les proportions molaires des isomères du diisocyanate de départ utilisé, par la nature chimique de la ou des amines utilisées dans le mélange réactionnel et/ou la proportion dans le mélange de départ, d'une amine par rapport à l'autre.

### EXEMPLE 1

Le mélange réactionnel contient 50 g (0,28 mole) de toluilène diisocyanate en mélange d'isomère 2,4 et d'isomère 2,6 en proportion 95/5 et un mélange d'amines constitué de 80 % molaire de 2-éthyl-héxylamine (m = 63,6 g et n = 0,49 mole) et de 20 % molaire de tertiobutyle amine (m = 9 g et n = 0,12 mole). La composition de la poudre blanche obtenue est déterminée par HPLC couplée à la spectrométrie de masse et contient bien le mélange de produits suivant dans les proportions attendues :

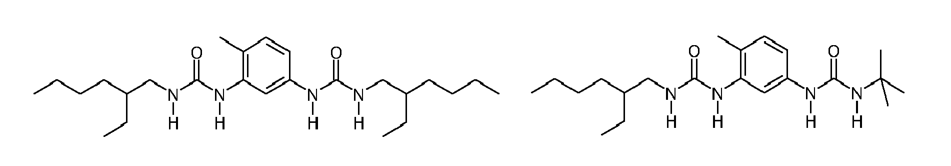

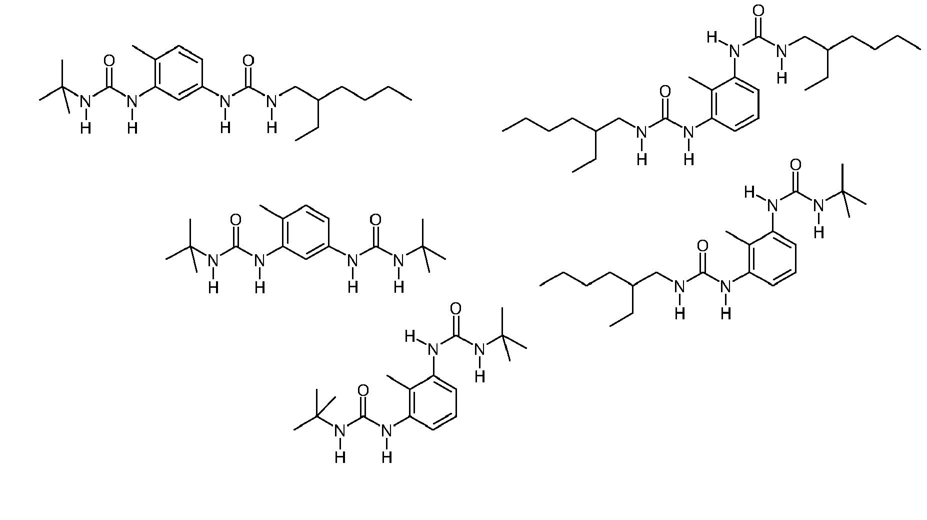

### EXEMPLE 2

Le mélange réactionnel contient du diisocyanate de toluène constitué d'un mélange d'isomères 2,4 et 2,6 en proportion 80/20 (m = 50 g et n = 0,28 mole) et un mélange d'amines constitué de 80 % molaire de 2-ethyl-hexylamine (m = 63,6 g et n = 0,49 mole) et de 20 % molaire de tertiobutyle amine (m = 9 g et n = 0,12 mole). La composition de la poudre blanche obtenue contient le même mélange de produits que celui obtenu à l'exemple 1 dans des proportions différentes (vérification par HPLC couplée à la masse).

### EXEMPLE 3

Le mélange réactionnel contient du diisocyanate de toluène en mélange d'isomère 2,4 et d'isomère 2,6 en proportion 95/5 (m = 15 g et n = 86 mmoles) et un mélange d'amines constitué de 80 % molaire d'2-ethyl hexylamine (m = 1,8 g et n = 139 mmoles) et de 20 % molaire de 3-(2-ethylhexyloxy)propylamine (m = 6,5 g et n = 35 mmoles). La composition de la poudre blanche obtenue est déterminée par HPLC couplée à la masse et contient dans les proportions attendues le mélange de produits suivant :

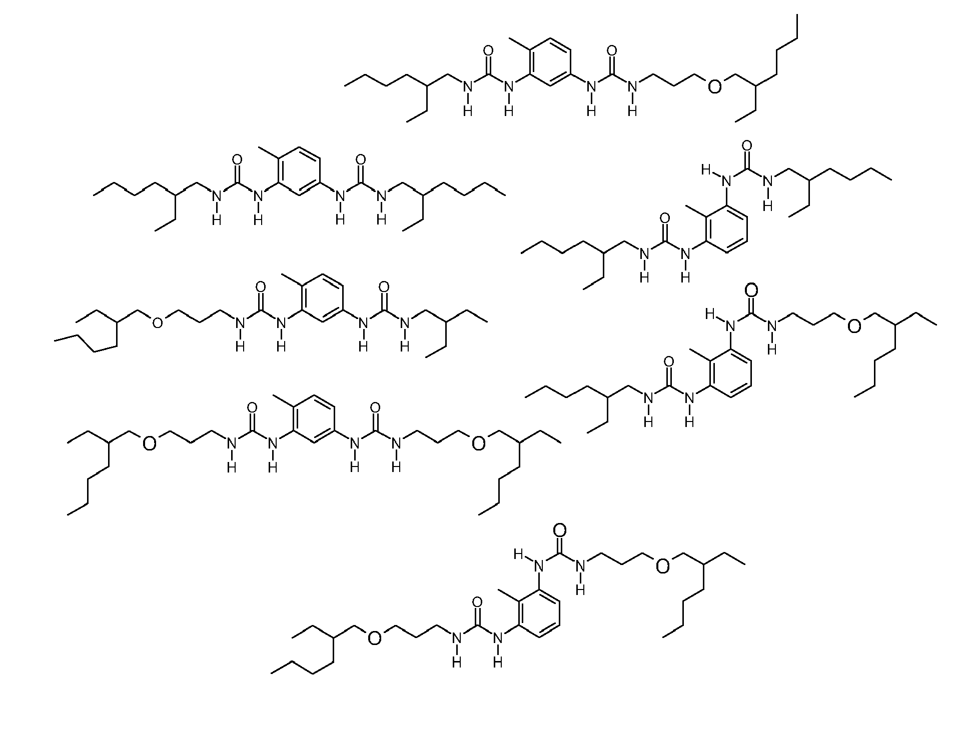

### EXEMPLE 4

Le mélange réactionnel contient du diisocyanate de toluène en mélange d'isomère 2,4 et d'isomère 2,6 en proportion 95/5 (m = 0,52 g et n = 3 mmoles) et un mélange d'amines constitué de 50 % molaire d'2-ethyl hexylamine (m = 0,39 g et n = 3 mmoles) et de 50 % molaire de 3-(2-éthylhexyloxy)propylamine (m = 0,56 g et n = 3 mmoles). La composition de la poudre blanche obtenue contient le même mélange de produits que celui obtenu à partir de l'exemple 3, mais dans des proportions différentes (Vérification par HPLC couplée à la masse).

### EXEMPLE 5

Le mélange réactionnel contient du diisocyanate de toluène en mélange d'isomère 2,4 et d'isomère 2,6 en proportion 95/5 (m = 0,72 g et n = 4,1 mmoles) et un mélange d'amines constitué de 50 % molaire de tertiobutylamine (m = 0,31 g et n = 4,2 mmoles) et 50 % molaire de 3-(2-ethylhexyloxy)propylamine (m = 0,79 g et n = 4,2 mmoles). La composition de la poudre blanche obtenue contient le mélange de produits suivant :

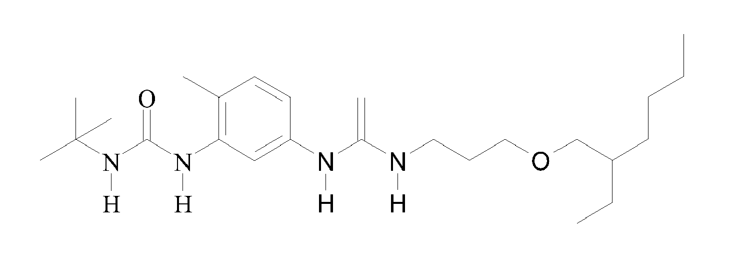

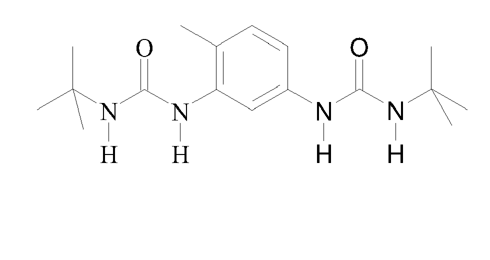

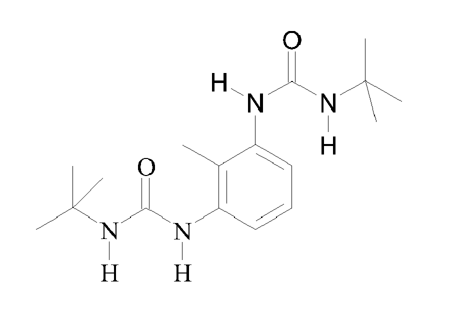

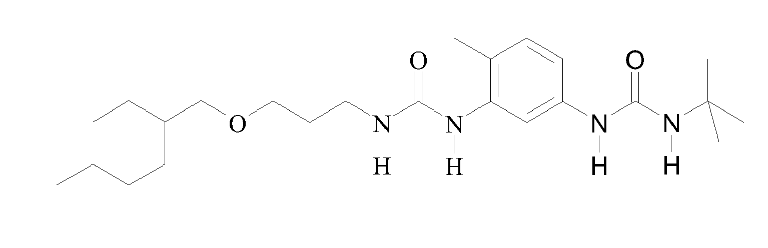

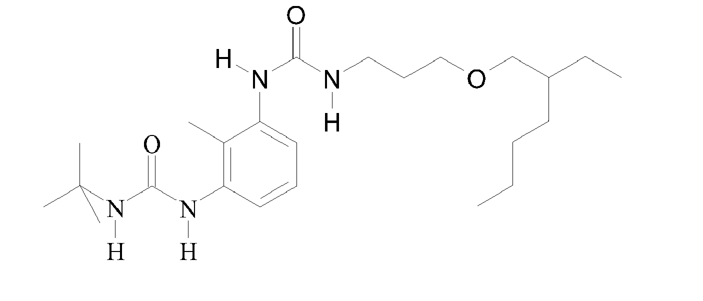

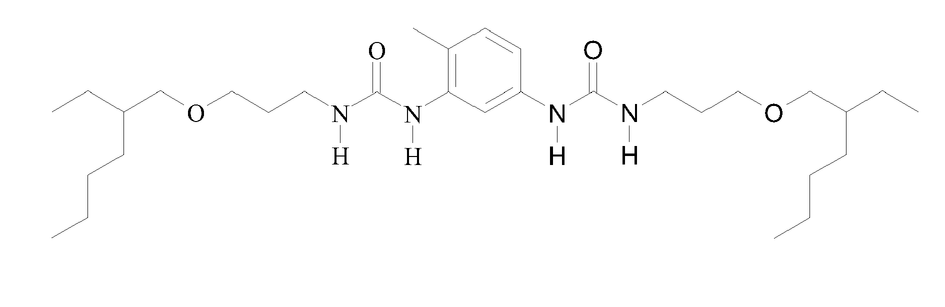

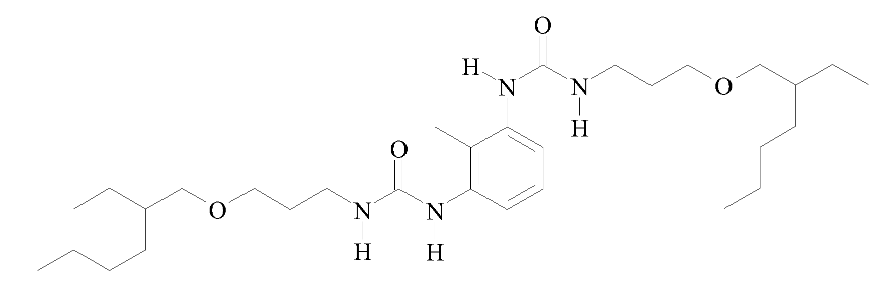

### EXEMPLE 6

Le mélange réactionnel contient du diisocyanate de toluène en mélange d'isomère 2,4 et d'isomère 2,6 en proportion 95/5 (m = 2,44 g et n = 14 mmoles) et un mélange d'amines constitué de 50 % molaire de 2-ethylhexylamine (m = 1,8 g et n = 14 mmoles) et de 50 % molaire de tertiobutylamine (m = 1,02 g et n = 14 mmoles). La composition de la poudre blanche obtenue contient le même mélange que celui de l'exemple 1 mais dans des proportions différentes confirmées par HPLC couplée à la masse.

### EXEMPLE 7

Le mélange réactionnel contient du diisocyanate de toluène en mélange d'isomère 2,4 et d'isomère 2,6 en proportion 95/5 (m = 50 g et n = 0,287 mole) et un mélange d'amines constitué de 50 % molaire de 2-ethylhexylamine (m = 40,8 g et n = 0,316 mole) et de 50 % molaire d'ethyl-3-aminobutirate (m = 41,4 g et n = 0,316 mole). La composition de la poudre blanche obtenue contient le mélange de produit suivant :

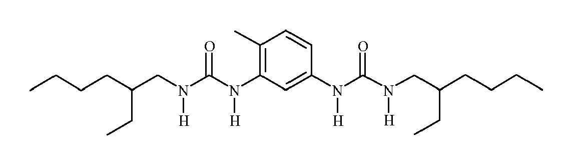

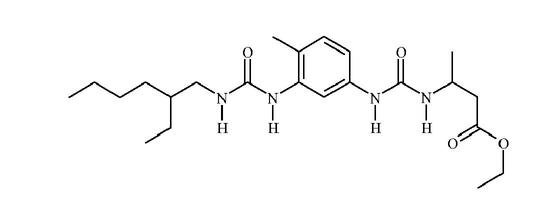

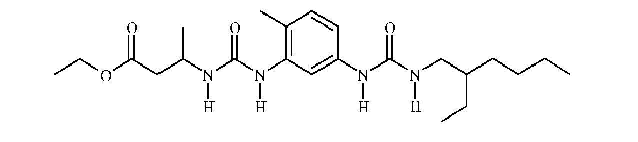

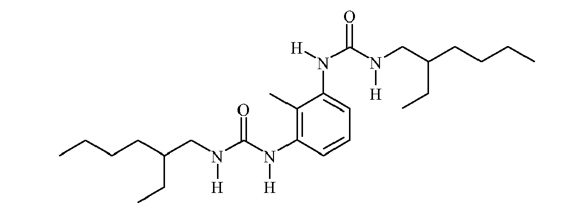

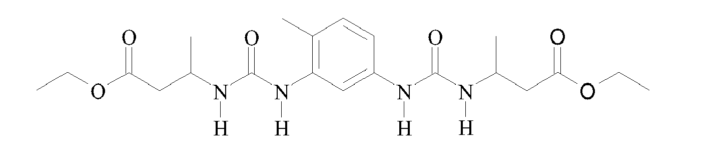

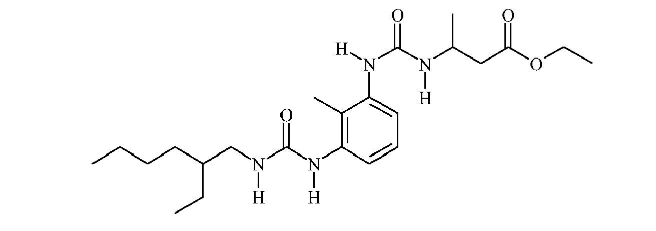

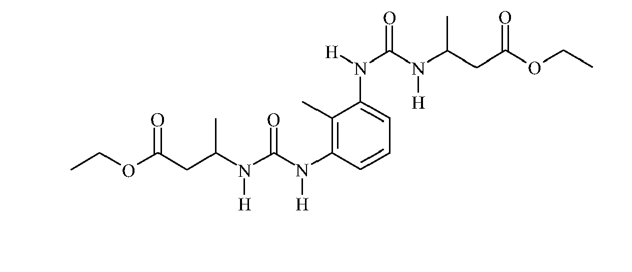

### EXEMPLE 8 : Exemple à partir d'un mélange à trois amines

Le mélange réactionnel contient du diisocyanate de toluène en mélange d'isomère 2,4 et d'isomère 2,6 en proportion 95/5 (m = 1,9 g ; n = 11 mmoles) et un mélange d'amines constitué de 80 % molaire de 2-ethylhexylamine(m = 2,2 g ; n = 17 mmoles), de 10% molaire de 3-(2-ethylhexyloxy)propylamine (m = 0,37 g ; n = 2 mmoles) et de 10 % molaire de tertiobutylamine (m = 0,15 g ; n = 2 mmoles).

### EXEMPLE 9 :

A titre comparatif, il a été préparé les dérivés symétriques suivants :

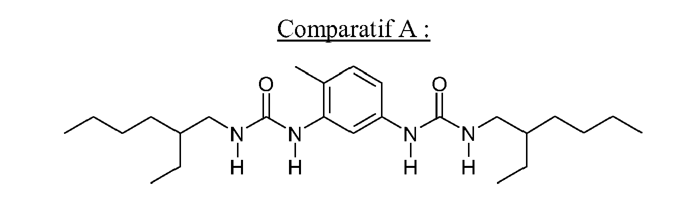

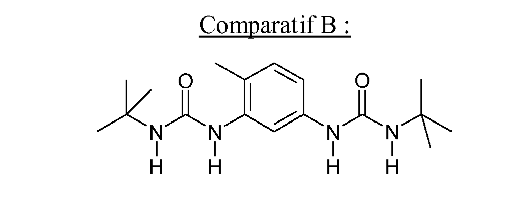

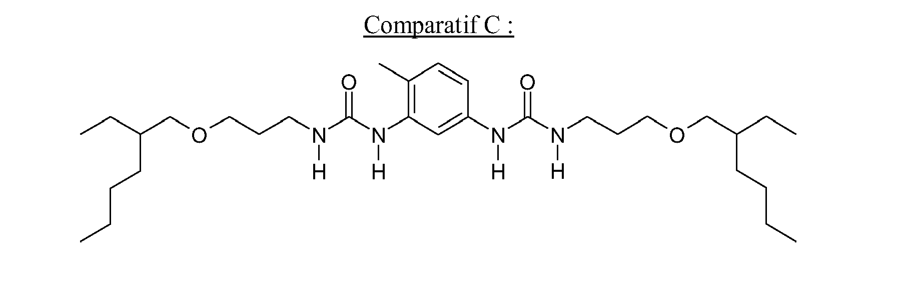

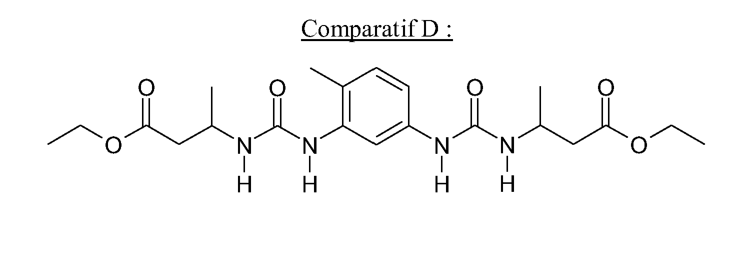

### EXEMPLE 10

Les mélanges de composés obtenus selon les exemples 1, 2, 3, 5 et 8 et les composés comparatifs A, B, C et D de l'exemple 9 sont testés à raison de 1 % et 10 % en poids pour leurs propriétés gélifiantes à température ambiante vis-à-vis d'une liste de solvants.

Pour ce faire, on introduit une quantité du composé ou mélange testé dans une quantité de solvant. Les propriétés gélifiantes sont jugées satisfaisantes si le composé ou mélange de composé se solubilise à température ambiante dans le solvant et, le cas échéant, s'il augmente la viscosité de ce dernier.

On note que seuls les mélanges des exemples 1, 2, 3, 5 et 8 comprenant un composé conforme à l'invention s'avèrent solubles à température ambiante à des concentrations jusqu'à 10 % en poids dans les phényltriméthicones et augmentent la viscosité de ces derniers.

En revanche, les dérivés symétriques des exemples comparatifs A, B et C ne s'avèrent pas solubles à 1% en poids dans la phényltriméthicone, même à 80 °C.

De la même manière, on observe que les mélanges des exemples 1, 2, 3, 5 et 8 sont solubles à température ambiante à 1 % en poids dans l'isododécane, dans l'octyldodécanol, dans l'isononanoate d'isononyle et dans l'huile de parléam et provoquent une augmentation sensible de la viscosité de ces derniers.

En revanche, les dérivés symétriques des exemples comparatifs A, B et C ne s'avèrent pas solubles à 1% en poids dans l'octyldodécanol, dans l'isononanoate d'isononyle et l'huile de parléam, même à 80 °C.

De plus, les mélanges de dérivés symétriques des exemples A, B, C et D ne sont pas solubles et ne provoquent pas une augmentation de la viscosité du milieu.

A titre d'exemple : exemple 1 est soluble à température ambiante à 1 % en poids dans l'isododécane, dans l'isononanoate d'isononyle et l'huile de parléam et provoque une augmentation de la viscosité du milieu. Par contre, nous n'observons pas ce résultat en mélangeant 80% en masse de l'exemple comparatif A avec 20% en masse de l'exemple comparatif B, (le mélange étant en tout à 1% massique dans le solvant), même en chauffant à 80°C.

Les mélanges des exemples 6 et 7 sont solubles à température ambiante à 3 % en poids dans l'acétate de butyle et provoque à cette concentration une augmentation de la viscosité de cette huile ester. On obtient les mêmes résultats avec le mélange de l'exemple 6 dans l'acétate d'éthyle.

En revanche, les dérivés symétriques des exemples comparatifs A, B et D de l'exemple 9 demeurent insolubles à 3 % en poids dans l'acétate d'éthyle et l'acétate de butyle.

### EXEMPLE 11

### Préparation d'un mascara

La composition est réalisée à partir des composés suivants :

| Nom | Concentration % massique |
|---|---|
| Cire d'Abeille | 9,9 |
| Cire de Carnauba | 4,52 |
| Paraffine | 2,18 |
| Polylaurate de vinyle¹ | 0,75 |
| Composé de l'Exemple 1 | 5 |
| Oxyde de fer noir | 2,5 |
| Bleu Outre mer | 2,1 |
| Conservateur | 0,2 |
| isododécane | Qsp |

| | |
|---|---|
| ¹ : Mexomère PP^{®} de CHIMEX | |

Un mascara aux qualités cosmétiques tout à fait satisfaisantes est obtenu.

## Revendications

1. Composition cosmétique à phase grasse liquide, comprenant dans un milieu physiologiquement acceptable au moins une phase grasse liquide, dans laquelle ladite phase est texturée par une quantité efficace d'au moins un composé de formule (I) : 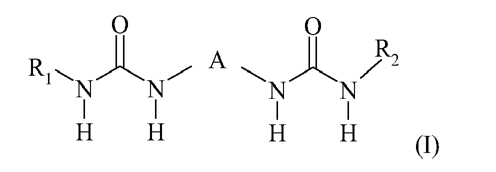 dans laquelle :
- A est un groupement de formule : 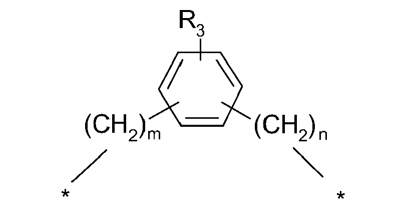 avec
- R₃ étant un atome d'hydrogène ou un radical alkyle en C₁ à C₄ linéaire ou ramifié,
- n et m étant, indépendamment l'un de l'autre, égaux à 0 ou 1, et
- * symbolisant le point de rattachement du groupement A aux deux atomes d'azote du reste du composé de formule générale (I),
- R₁ est un radical alkyle en C₃ à C₁₅, ramifié, non cyclique, saturé ou insaturé et contenant éventuellement de 1 à 3 hétéroatomes choisis parmi O, S, F et N, et/ou un carbonyle et leurs combinaisons,
- R₂ est différent de R₁ et est choisi parmi les radicaux alkyles en C₁-C₂₄, saturés ou insaturés, linéaires, ramifiés ou cycliques, contenant éventuellement de 1 à 3 hétéroatomes choisis parmi O, S, F et N, et éventuellement substitué par :
- 1, 2 ou 3 radicaux hydroxy,
- un radical ester (-COOR₄), avec R₄ étant un radical alkyle, linéaire
ou ramifié, ayant de 1 à 8, notamment 1 à 6 voire 2 à 4 atomes de carbone,
- un radical cyclique saturé, insaturé ou aromatique ayant de 5 à 12 atomes de carbone, en particulier un radical phényle éventuellement substitué par un ou plusieurs radicaux identiques ou différents choisis parmi alkyle C₁-C₄, trifluorométhyle, ou un dérivé morpholine, et/ou
- un ou plusieurs radicaux alkyles linéaires ou ramifiés en C₁-C₄,
ou l'un de ses sels ou isomères.

2. Composition selon la revendication 1, dans laquelle le groupement A est un groupement de formule : 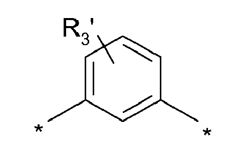 avec R₃' étant un radical alkyle en C₁ à C₄ linéaire ou ramifié et * symbolisant les point de rattachement du groupement A aux deux atomes d'azote du reste du composé de formule générale (I).

3. Composition selon la revendication précédente, dans laquelle le groupement A est un groupement de formule : 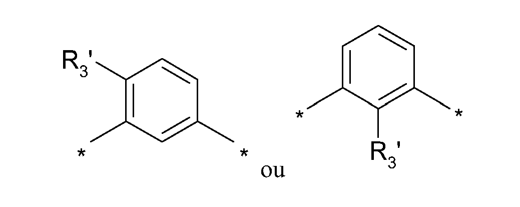 pur ou en mélange avec les autres isomères de position, notamment dans les proportions 95/5 ou 80/20 avec R₃' et * étant tels que définis en revendication 2.

4. Composition selon la revendication précédente, dans laquelle le groupement A est un groupement de formule : 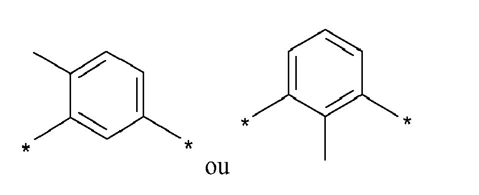 * étant tel que défini en revendication 2.

5. Composition selon l'une quelconque des revendications précédentes, dans laquelle le groupement R₁ est choisi parmi : 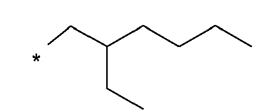 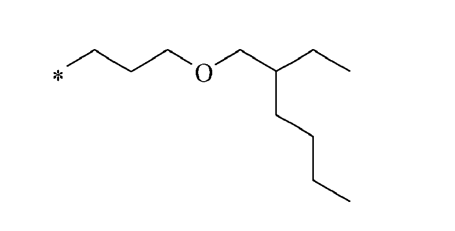 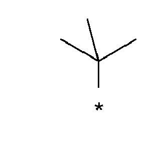 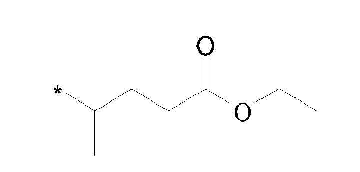 avec * symbolisant le point de rattachement du groupement R₁ à l'azote du reste du composé de formule générale (I).

6. Composition selon l'une quelconque des revendications précédentes, dans laquelle le groupement R₂ est choisi parmi : 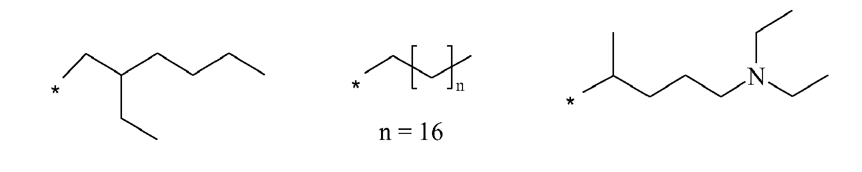 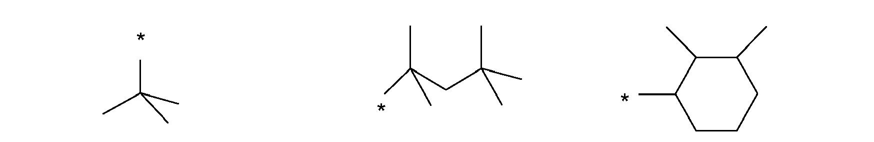 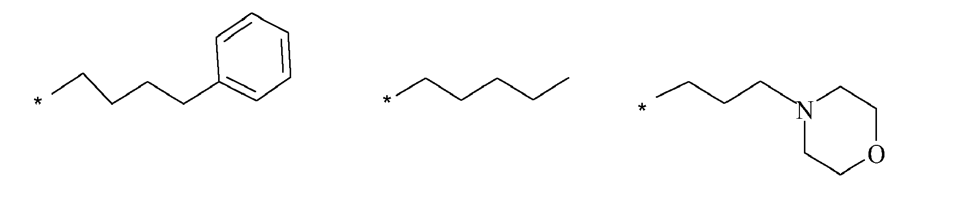 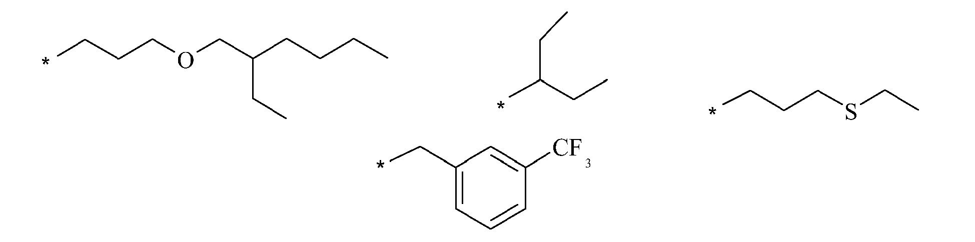 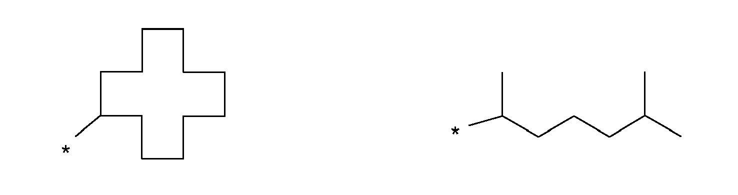 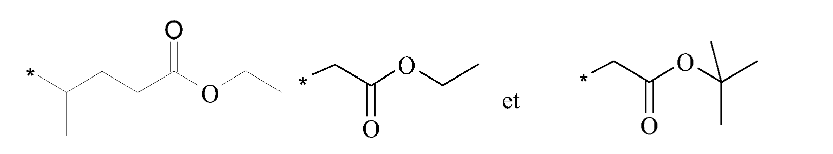 avec * symbolisant le point de rattachement du groupement R₂ à l'azote du reste du composé de formule générale (I).

7. Composition selon l'une quelconque des revendications précédentes, dans laquelle le composé de formule (I) est choisi parmi : 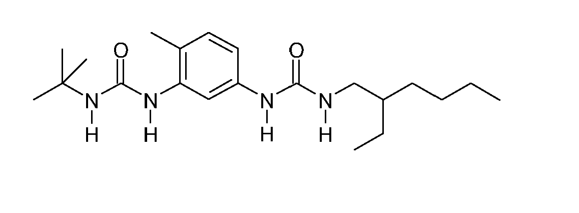 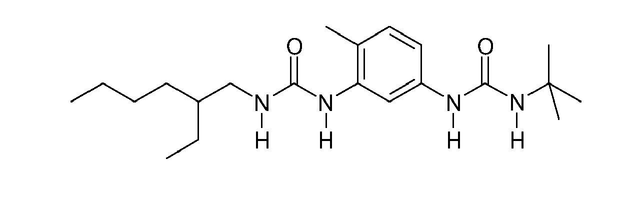 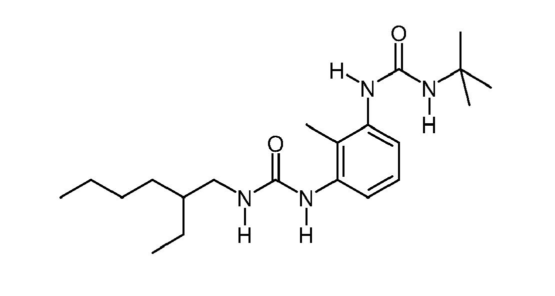 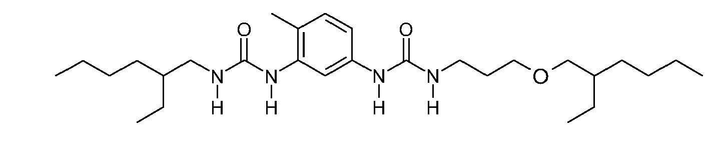 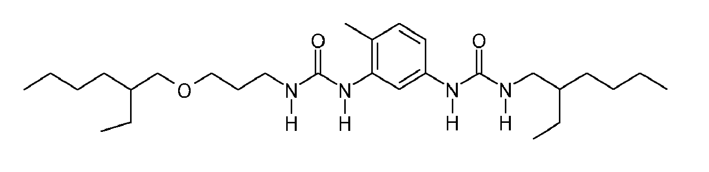 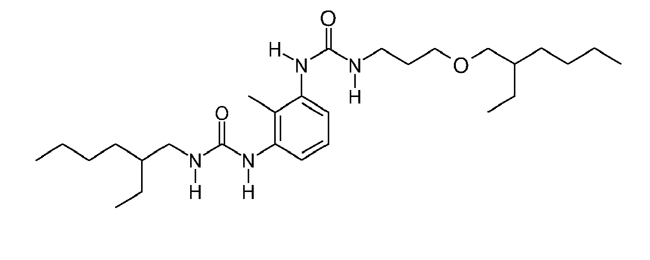 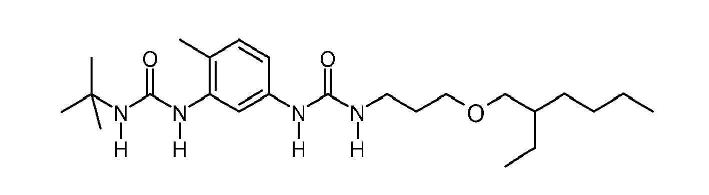 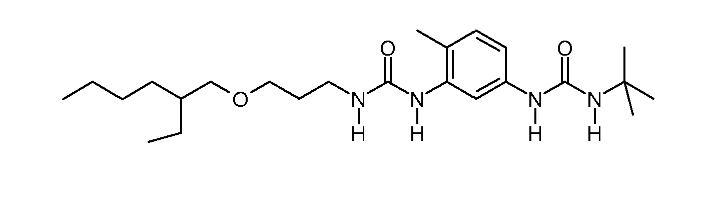 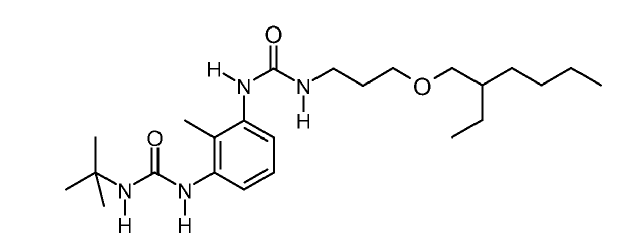 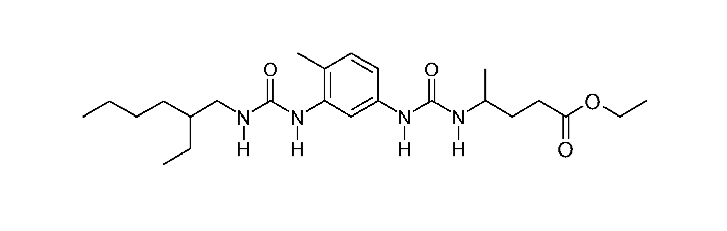 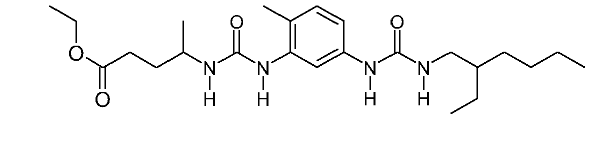 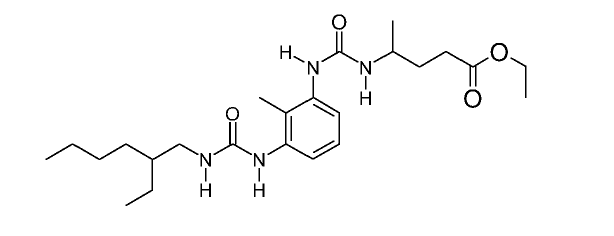 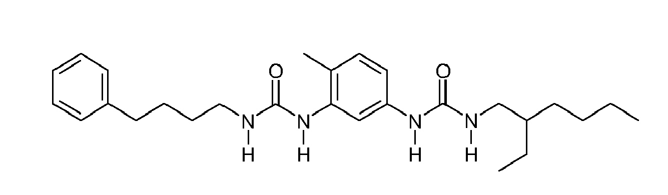 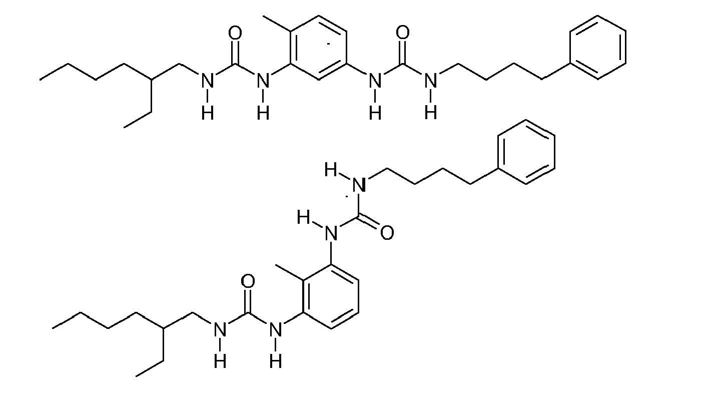 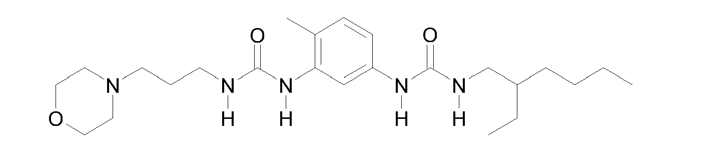 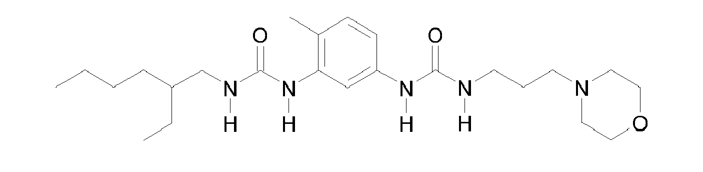 et

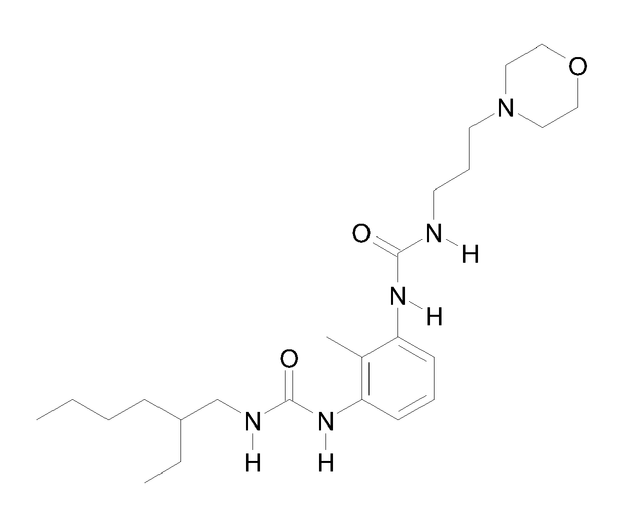

8. Composition selon l'une quelconque des revendications précédentes, dans laquelle l'agent gélifiant comprend en outre au moins un composé de formule (II) : 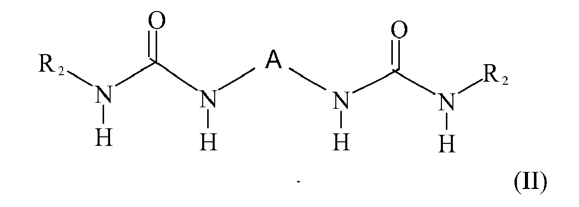 avec A et R₂ tels que définis selon l'une quelconque des revendications 1 à 4 et 6, A et R₂ ayant la même définition en formules (I) et (II).

9. Composition selon la revendication précédente, dans laquelle l'agent gélifiant comprend en outre au moins un composé de formule (III) : 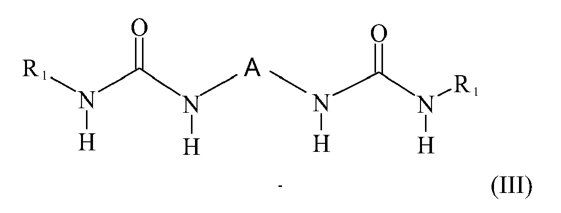 avec A et R₁ tels que définis selon l'une quelconque des revendications 1 à 5, R₁ ayant la même définition en formules (I) et (III) et A ayant la même définition en formules (I), (II) et (III).

10. Composition selon l'une quelconque des revendications précédentes, dans laquelle la phase grasse liquide comprend au moins une huile siliconée choisie parmi : les polyalkylméthylsiloxanes et en particulier des polydiméthylsiloxanes comportant des groupements alkyle, alcoxy ou phényle, pendants et/ou en bout de chaîne siliconée, groupements ayant chacun de 2 à 24 atomes de carbone ; les diméthicone copolyols, les alkylméthicone copolyols ; des silicones phénylées comme les phényl triméthicones, les phényl diméthicones, les phényl triméthylsiloxy diphénylsiloxanes, les diphényl diméthicones, les diphényl méthyldiphényl trisiloxanes, des 2-phényléthyl triméthylsiloxysilicates, la cétyldiméthicone, les silicones à groupes alkylglycéryl éthers, les silicones à groupes amines latéraux et le dilauroyltriméthylol propane siloxysilicate ; des silicones fluorées à groupement(s) pendant(s) ou en bout de chaîne ayant de 1 à 12 atomes de carbone dont tout ou partie des atomes d'hydrogène est substitué par des atomes de fluor telles que les silicones à groupes alkyle et perfluoralkyle, les silicones à groupes latéraux oxyéthylénés/oxypropylénés (OE/PP) et à groupes perfluorés, les silicones à groupes latéraux perfluorés et à groupes latéraux glycérolés, les perfluoroalkylméthylphénylsiloxanes, et leurs mélanges.

11. Composition selon l'une quelconque des revendications précédentes, dans laquelle la phase grasse liquide comprend au moins une huile ester choisie parmi les esters de formule (IV) suivante :
R₁-CO-O-R₂ (IV)
dans laquelle :
- R₁ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substituées, et
- R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

12. Composition selon l'une quelconque des revendications précédentes, dans laquelle la phase grasse liquide comprend au moins une huile hydrocarbonée ou un éther volatile choisi parmi les huiles végétales hydrocarbonées, les éthers de synthèse en C₆-C₄₀, les alcools gras en C₈-C₃₂, les acides gras en C₈-C₃₂, et leurs mélanges, les huiles de paraffine, les polyisobutènes hydrogénés et/ou leur mélanges.

13. Composition selon l'une quelconque des revendications précédentes, dans laquelle la phase grasse liquide comprend au moins une huile choisie parmi : l'octyldodécanol, le Parléam, l'isododécane, l'isononanoate d'isononyle, une phényltriméticone, le N-lauroyl sarcosinate d'isopropyle (Eldrew 205), l'acétate d'éthyle, l'acétate de butyle et/ou leurs mélanges.

14. Composition selon l'une quelconque des revendications précédentes, dans laquelle ledit composé de formule générale (I) dérive de la réaction entre au moins un diisocyanate de formule : 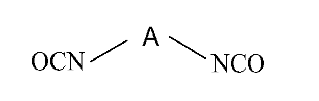 et au moins deux amines primaires distinctes de formules : 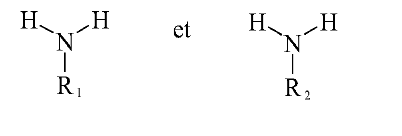 avec A, R₁ et R₂ tels que définis selon l'une quelconque des revendications 1 à 6.

15. Composition selon la revendication précédentes, dans laquelle le nombre d'amines primaires utilisés est de deux et le rapport molaire n(R₁)/n(R₂) varie entre 10/90 et 90/10
avec n(R₁) correspondant au nombre de moles de : 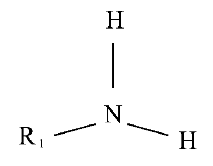 et n(R₂) correspondant au nombre de moles de : 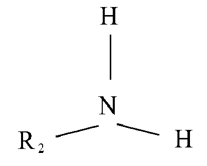 avec R₁ et R₂ tels que définis selon l'une quelconque des revendications 1 à 6.

16. Composition selon la revendication 14 ou 15, dans laquelle le nombre d'amines utilisées pour la réaction est supérieur ou égal à 2, par exemple varie de 2 à 20, et plus particulièrement entre 3 et 10.

17. Composition selon l'une quelconque des revendications 14 à 16, dans laquelle l'ensemble des amines utilisées est dans un rapport molaire de 2 à 3 équivalents pour un équivalent de diisocyanate.

18. Composition selon l'une quelconque des revendications précédentes, comprenant moins de 0,5 % en poids en composé(s) de formule générale (I) par rapport au poids total de la phase grasse liquide.

19. Composition selon l'une quelconque des revendications précédentes, comprenant en outre au moins une matière colorante.

20. Composition selon l'une quelconque des revendications précédentes, comprenant en outre au moins une charge.

21. Composition selon l'une quelconque des revendications précédentes, contenant en outre, au moins un actif cosmétique ou dermatologique.

22. Composition selon l'une quelconque des revendications précédentes, contenant en outre au moins un additif choisi parmi l'eau, les vitamines, les épaississants distincts de ceux répondant à la formule générale (I), les gélifiants distincts de ceux répondant à la formule générale (I), les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

23. Utilisation d'un composé de formule générale (I) tel que défini selon l'une des revendications précédentes pour structurer une composition cosmétique.

24. Procédé de soin et/ou de maquillage d'une matière kératinique, notamment de la peau, comprenant l'application sur la surface à traiter d'une composition selon l'une quelconque des revendications 1 à 22.
